# EUROPÄISCHE PATENTANMELDUNG

(11) **EP 1 602 358 A1**
(43) Veröffentlichungstag der Anmeldung: **07.12.2005**
(21) Anmeldenummer: 05010467.8
(22) Anmeldetag: 03.04.2000
(51) Int. Cl.: A61K 7/48

(54) **Kosmetische Formulierung enthaltend eine Pigmentmischung**

(30) Priorität: 16.04.1999 DE 19917388
(62) Teilanmeldung aus: 00107050.7
(71) Anmelder: Merck Patent GmbH, 64293 Darmstadt (DE)
(72) Erfinder: Pfaff, Gerhard, Dr., 64839 Münster (DE); Schoen, Sabine, Dr., 45701 Herten (DE); Anselmann, Ralf, Dr., 59348 Luedinghausen-Seppenrade (DE)

(57) **Zusammenfassung**

Die vorliegende Erfindung betrifft kosmetische Formulierungen enthaltend Pigmentmischungen bestehend aus mindestens zwei Komponenten, wobei Komponente A Mehrschichtpigmente (Multischichtpigmente) auf Basis von Glimmer, SiO₂-Plättchen, Glasplätichen, Al₂O₃-Plättchen oder Polymerplättchen und Komponente B plättchenförmige, nadelförmige oder sphärische Farbmittel oder Füllstoffe sind.

## Beschreibung

Die vorliegende Erfindung betrifft kosmetische Formulierungen enthaltend Pigmentmischungen bestehend aus mindestens zwei Komponenten, wobei Komponente A Mehrschichtpigmente (Multischichtpigmente) auf Basis von Glimmer, SiO₂-Plättchen, Glasplättchen, Al₂O₃-Plättchen oder Polymerplättchen und Komponente B plättchenförmige, nadelförmige oder sphärische Farbmittel und/oder Füllstoffe sind.

Deckvermögen und Glanz sind bei plättchenförmigen Pigmenten oftmals nur schwer gleichzeitig in befriedigendem Ausmaß zu realisieren. So zeichnen sich etwa mit einer oder mehreren dünnen Metalloxidschichten belegte Glimmerplättchen oder SiO₂-Plättchen durch Interferenzfarben und einen hohen Glanz, gleichzeitig aber auch wegen des durchsichtigen Substrats durch eine hohe Transparenz und damit ein vergleichsweises geringes Deckvermögen, aus.

Aus der DE-A-42 40 511 ist eine Pigmentmischung bekannt, die sich aus einem Interferenzpigment und einem plättchenförmigen Farbpigment zusammensetzt. Das Interferenzpigment sind mit Metalloxiden beschichtete Glimmer oder SiO₂-Plättchen und das Farbpigment können farbige SiO₂-Plättchen sein. Dieses Pigmentgemisch wird in Lacke, Druckfarben, Kunststoffe oder Kosmetika, eingearbeitet

Aufigabe der vorliegenden Erfindung ist es eine Pigmentmischung bereit zustellen, die sich durch ein vergleichsweise hohes Deckvermögen auszeichnet und sich gut in das jeweilige Anwendungssystem einarbeiten läßt und bei der gleichzeitig eine Trennung Pigment/Farbmittel im System weitgehend ausgeschlossen ist.

Überraschenderweise wurde nun eine Pigmentmischung gefunden, die keine der oben angegebenen Nachteile aufweist Das erfindungsgemäße Pigmentgemisch besteht aus mindestens zwei Komponenten, wobei Komponente A Mehrschichtpigmente (Multischichtpigmente) auf Basis von Glimmer, SiO₂-Plättchen, Glasplättchen, Al₂O₃-Plättchen oder Polymerplättchen, und Komponente B plättchenförmige, nadelförmige oder sphärische Farbmittel und/oder Füllstoffe sind.

Durch die Zumischung eines oder mehrerer Farbmittel zu den Mehr schichtpigmenten (Multischichtpigmenten) kann den Anwendungssystemen ein Farbflop (stark winkelabhängiger Farbeindruck) verliehen werden, der Farbeffekt wird verstärkt und neuartige Farbeffekte werden erzielt.

Gegenstand der Erfindung ist somit eine kosmetische Formulierung enthaltend ein Pigmentgemisch bestehend aus mindestens zwei Komponenten, wobei Komponente A Mehrschichtpigmente (Multischichtpigmente) auf Basis von Glimmer, SiO₂-Plättchen, Glasplättchen, Al₂O₃-Plättchen oder Polymerplättchen, und Komponente B plättchenförmige, nadelförmige oder sphärische Farbmittel und/oder Füllstoffe sind.

Die Mehrschichtpigmente können in jedem Verhältnis mit dem Farbmittel bzw. Füllstoff gemischt werden. Vorzugsweise ist das Verhältnis von Komponente A zu Komponente B1 : 20 bis 20 : 1, insbesondere 1:10 bis 10:1.

Die beispielsweise aus den deutschen Offenlegungsschriften
DE 196 18 563, DE 196 18 566, DE 196 18 569, DE 197 07 805,
DE 197 07 806, DE 197 46 067 bekannten Mehrschichtpigmente basieren auf einer plättchenförmigen, transparenten, farbigen oder farblosen Matrix, bestehend aus Glimmer (synthetisch oder natürlich), SiO₂-Plättchen, Glasplättchen, Al₂O₃-Plättcheil, Polymerplättchen, und besitzen in der Regel eine Dicke zwischen 0,3 und 5 µm, insbesondere zwischen 0,4 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die Mehrschichtpigmente bestehen aus der Matrix (Substrat) beschichtet mit Metalloxiden (mindestens 2). Die Beschichtung der Substratplättchen Glimmer, SiO₂-Piättchen, Glasplättchen, Al₂O₃-Plättchen mit mehreren Schichten erfolgt so, daß ein Schichtaufbau bestehend aus alternierenden hoch- und niedrigbrechenden Schichten entsteht Vorzugsweise enthalten die Mehrschichtpigmente 2, 3, 4, 5, 6 oder 7 Schichten, insbesondere 3, 4 oder 5 Schichten. Geeignete hochbrechende Metalloxide sind beispielsweise Titandioxid, Zirkonoxid, Zinkoxid, Eisenoxide. Eisen-Titan-Oxide (Eisentitanate) und/oder Chromoxid, insbesondere TiO₂ und/oder Fe₂O₃. Als niedrigbrechende Metalloxide kommen SiO₂ und Al₂O₃ zum Einsatz. Es kann hierfür jedoch auch MgF₂ oder ein organisches Polymer (z.B. Acrylat) eingesetzt werden. Die Beschichtung der Substratplättchen kann z.B, erfolgen wie in der WO 93/08237 (naßchemische Beschichtung) oder DE-OS-196 14 637 (CVD-Verfahren) beschrieben.

Bevorzugte Mehrschichtpigmente besitzen folgenden Aufbau:

Substrat + Fe₂O₃-Schicht + SiO₂-Schicht + Fe₂O₃-Schicht

Substrat + Fe₂O₃-Schicht + SiO₂-Schicht + TiO₂-Schicht

Substrat + TiO₂-Schicht + SiO₂-Schicht + Fe₂O₃-Schicht

Substrat + TiO₂-Schicht + SiO₂-Schicht + TiO₂/Fe₂O₃-Schicht

Substrat + TiO₂/Fe₂O₃-Schicht + SiO₂-Schicht + TiO₂/Fe₂O₃-Schicht

Substrat + TiO₂-Schicht + SiO₂-Schicht + Cr₂O₃-Schicht

Anstelle der äußeren Metalloxidschicht kann auch eine semitransparente Schicht eines Metalls verwendet werden. Geeignete Metalle dafür sind beispielsweise Cr, Ti, Mo, W, Al, Cu, Ag, Au oder Ni.

Zur Erzielung spezieller Farbeffekte können in die hoch- bzw. niedrigbrechenden Schichten zusätzlich noch feinteilige Partikel im Nanometergrößenbereich eingebracht werden. Als geeignet dafür erweisen sich beispielsweise feinteiliges TiO₂ oder feinteiliger Kohlenstoff (Ruß) mit Teilchengrößen im Bereich von 10-250 nm. Durch die lichtstreuenden Eigenschaften derartiger Partikel kann gezielt auf Glanz und Deckvermögen Einfluß genommen werden.

Die Mehrschichtpigmente können auch zur Verbesserung der Licht-, Wetter- und chemischen Stabilität oder zur Erhöhung der Kompatibilität in unterschiedliche Medien noch mit einer Schutzschicht versehen sein. Als Nachbeschichtungen bzw. Nachbehandlungen kommen beispielsweise die in den DE 22 15 191, DE 31 51 354, DE 32 35 017 oder DE 33 34 598 beschriebenen Verfahren in Frage. Die zusätzlich aufgebrachten Stoffe machen nur etwa 0,1 bis 5 Gew.-%, vorzugsweise 0,5 bis 3,0 Gew.-%, des Mehrschichtpigments aus.

Als Komponente B für die erfindungsgemäße Pigmentmischung sind alle dem Fachmann bekannten plättchenförmigen, nadelförmigen und sphärischen Farbmittel oder Füllstoffe geeignet, die eine Partikelgröße von 0,001 bis 10 µm, vorzugsweise 0,01 bis 1 µm, aufweisen. Bevorzugt enthalten die erfindungsgemäßen Pigmentgemische als Farbmittel Absorptionspigmente und als Füllstoffe plättchenförmige oder sphärische Pulver. Komponente B sind vorzugsweise beschichtete oder unbeschichtete SiO₂-Kugeln. Mit ein oder mehreren Metalloxiden beschichtete SiO₂-Kugeln sind z.B. aus der EP 0 803 550 A2 bekannt.

Bevorzugte Pigmentgemische enthalten neben Komponente A als Farbmittel (Komponente B) insbesondere ein Perlglanzpigment. Als Pedglanzpigmente werden Pigmente auf der Basis plättchenförmiger, transparenter oder semitransparenter Substrate aus z.B. Schichtsilikaten, wie etwa Glimmer, synthetischem Glimmer, Talkum, Sericit, Kaolin, aus Glas oder anderen silikatischen Materialien verwendet, die mit farbigen oder farblosen Metalloxiden wie z.B. TiO₂, Titansuboxide, Titanoxinitride, Fe₂O₃, Fe₃O₄, SnO₂, Cr₂O₃, ZnO, CuO, NiO und anderen Metalloxiden allein oder in Mischung in einer einheitlichen Schicht oder in aufeinanderfolgenden Schichten beschichtet sind. Perlglanzpigmente sind z.B, aus den deutschen Patenten und Patentanmeldungen 14 67 468, 19 59 998, 20 09 566, 22 14 454, 22 15 191, 22 44 298, 23 13 331, 25 22 572, 31 37 808, 31 37 809,31 51 343, 31 51 354, 31 51 355, 32 11 602, 32 35 017 und P 38 42 330 bekannt und im Handel erhältlich, z.B. unter der Marke Iriodin® der Firma Merck KGaA, Darmstadt, Deutschland. Besonders bevorzugte Pigmentpräparationen enthalten TiO₂(Glimmer-, Fe₂O₃/Glimmer- und/oder TiO₂/Fe₂O₃-Glimmerpigmente.

Weiterhin bevorzugt sind mit TiO₂ und/oder Fe₂O₃ beschichtete SiO₂- oder Al₂O₃-Plättchen, Die Beschichtung der SiO₂-Plättchen mit ein oder mehreren Metalloxiden kann z.B. erfolgen wie in der WO 93/08237 (naßchemische Beschichtung) oder DE-OS 196 14 637 (CVD-Verfahren) beschrieben.

Als plättchenförmige Farbmittel kommen vor allem Perlglanzpigmente insbesondere auf Basis von Glimmer, SiO₂-Plättchen, Al₂O₃-Plättchen oder Glas-Plättchen, die nur mit einer Metalloxidschicht umhüllt sind, Metalleffektpigmente (Al-Plättchen, Bronzen), optisch variable Pigmente (OVP's), Flüssigkristallpolymerpigmente (LCP's) oder holographische Pigmente in Frage.

Zu den sphärischen Farbmitteln zählen insbesondere TiO₂, eingefärbtes SiO₂, CaSO₄, Eisenoxide, Chromoxide, Ruß, organische Farbpigmente, wie z.B. Anthrachinon-Pigmente, Chinacridon-Pigmente, Diketopyrrolopyrrol-Pigmente, Phthalocyanin-Pigmente, Azopigmente, Isoindolin-Pigmente. Bei den nadelförmigen Pigmenten handelt es sich vorzugsweise um BiOCl, eingefärbte Glasfasern, α-FeOOH, organische Farbpigmente, wie z.B. Azopigmente, β-Phthatocyanin Cl Blue 15,3, Cromophtalgelb 8GN (Ciba-Geigy), Irgalith Blau PD56 (Ciba-Geigy), Azomethinkupferkomplex Cl Yellow 129, Irgazingelb 5GT (Ciba-Geigy).

Die erfindungsgemäße Pigmentmischung für kosmetische Formulierungen ist einfach und leicht zu handhaben. Die Pigmentmischung kann durch einfaches Einrühren in das Anwendungssystem eingearbeitet werden. Ein aufwendiges Mahlen und Dispergieren der Pigmente ist nicht erforderlich.

Die erfindungsgemäße Pigmentmischung kann zur Pigmentierung von kosmetischen Formulierungen, wie Lippenstifte, Nagellacke, Preßpuder, Shampoos, lose Puder und Gele, verwendet werden. Die Konzentration der Pigmentmischung im zu pigmentierenden Anwendungssystem liegt in der Regel zwischen 0,1 und 70 Gew.%, vorzugsweise zwischen 0,1 und 50 Gew.% und insbesondere zwischen 1,0 und 10 Gew.%, bezogen auf den Gesamtfestkörpergehalt des Systems. Sie ist in der Regel abhängig vom konkreten Anwendungsfall.

Das erfindungsgemäße Pigmentgemisch kann vorteilhaft in der dekorativen und pflegenden Kosmetik eingesetzt werden. Die Einsatzkonzentralen und das Mischungsverhältnis von Mehrschichtpigmenten mit Komponente B, insbesondere organischen und anorganischen Farbpigmenten und Farbstoffen, natürlichen oder synthetischen Ursprungs, wie z.B. Chromoxid, Ultramarin, sphärischen SiO₂- oder TiO₂-Pigmenten, sind abhängig vom Anwendungsmedium und dem Effekt, der erzielt werden soll. Die Mischung aus Mehrschichtpigmenten mit anderen Pigmenten oder Farbstoffen kann in allen Verhältnissen erfolgen, vorzugsweise beträgt das Verhältnis 1 : 10 bis 10 : 1. Die Einsatzkonzentration reicht von 0,01 Gew.% im Shampoo bis zu 70 Gew. % im Preßpuder. Bei einer Mischung von Mehrschichtpigmenten mit sphärischen Füllstoffen, z. B. SiO₂, kann die Konzentration bei 0,01-70 Gew.% in der Formulierung liegen. Die kosmetischen Produkte, wie z.B. Nagellacke, Lippenstifte, Preßpuder, Shampoos, lose Puder und Gele, zeichnen sich durch besonders interessante Glanz- und/oder Farbeffekte aus, die je nach Aufbau des Mehrschichtpigments stark winkelabhängig sein können. Der Glitzereffekt in Nagellack kann gegenüber herkömmlichen Nagellacken mit Hilfe der erfindungsgemäßen Pigmentgemische deutlich gesteigert werden. Weiterhin kann das erfindungsgemäße Pigmentgemisch in Badezusätzen, Zahnpasten eingesetzt werden.

Die nachfolgenden Beispiele sollen die Erfindung erläutern, ohne sie jedoch zu begrenzen.

### Beispiele

| Beispiel 1: - Lidschatten | |
|---|---|
| Phase I | |
| 5,00 % | Silica |
| 25,00 % | SiO₂-Plättchen der Teilchengröße 5-40 µm beschichtet mit 3 Schichten in der Folge Fe₂O₃, SiO₂, Fe₂O₃ |
| 5,00 % | C.I. Pigment Green 18 (CI77289) |
| 47,42 % | Talc |
| 7,18% | Solanum Tuberosum (Potato Starch) |
| 2.40% | Magnesium Stearate |

| Phase II | |
|---|---|
| 6,96 % | Isopropyl Stearate |
| 0,40 % | Cetyl Palmitate |
| 0,40 % | Petrolatum |
| 0,08 % | Konservierung |

Die Bestandteile der Phase I werden zusammengegeben und vorgemischt. Anschließend wird die Pudermischung unter Rühren tropfenweise mit der geschmolzenen Phase II versetzt. Die Puder werden bei 40-50 bar gepreßt

Der erhaltene Lidschatten zeichnet sich durch einen intensiven Glanz und einen stark changierenden Farbeindruck je nach Blickwinkel aus.

| Beispiel 2: - Lippenstift | |
|---|---|
| Phase I | |
| 8,25 % | Cera alba |
| 4,95% | Ceresin, Copemica Cerifera |
| 3,30% | Lanolin Oil |
| 5,28 % | Isopropyl Myristate |
| 1,98% | Mineral Oil |
| 0,03% | Tocapherol, Ascorbyl Palmitate, Ascorbic Acid, Citric Acid, PEG-8 |
| 0,06 % | Konservierung |
| 0,50 % | Aroma |
| 1,00 % | Lithalrubin BK, C.I. Pigment Red 57:1 (CI 15850) |
| ad 100,00 % | Ricinus Communis (20 % in Castor Oil) |

| Phase II | |
|---|---|
| 2.00 % | Silica |
| 15.00 % | SiO₂-Plättchen der Teilchengröße 5-40 µm beschichtet mit 3 Schichten in der Folge Fe₂O₃, SiO₂, Fe₂O₃ |

Die Bestandteile der Phase I werden auf 75°C erhitzt und aufgeschmolzen. Die Pigmente der Phase II werden zugegeben und alles gut durchgerührt. Die Lippenstiftmasse wird dann in der auf 65 °C temperierten Gießapparatur 15 Min. gerührt. Die homogene Schmelze wird in die auf 65 °C vorgewärmten Gießformen gegossen. Anschließend kühlt man die Formen ab und entfernt die Gießlinge kalt.

Der Lippenstift zeichnet sich durch einen intensiven Glanz und einen stark changierendert Farbeindruck je nach Blickwinkel aus.

| Beispiel 3: - Duschgel | |
|---|---|
| Phase A | |
| 0,10 % | Glimmerpigment der Teitchengröße 10-60 µm, beidseitig beschichtet mit TiO₂, SiO₂, TiO₂ (1) |
| 0,75 % | Xanthan Gum (2) |
| 62,10% | Wasser |

| Phase B | |
|---|---|
| 20,00 % | Decyl Glucoside (3) |
| 6,65 % | Texapon ASV (Sodium Laureth Sulfate, Magnesium Laureth Sulfate, Sodium Laureth 8-Sulfate, Magnesium Laureth 8-Sulfate, Sodium Oleth Sulfate, Magnesium Oleth Sulfate (3) |
| 0,20 % | Bronidox L (Propylene Glycol, 5-Bromo-5-nitro-1,3-dioxane)(3) |
| 0,05 % | Parfüm (Parfümöl Everest 79658 SB) (4) |

| Phase C | |
|---|---|
| 0,15 % | Citric Acid (1) |
| 0,10% | Sicovit Grün Z 6120 (1 %ig in Wasser) [Cl 19140/42051] (5) |
| 10,00 % | Wasser |

| | |
|---|---|
| Bezugsquellen: (1) Merck KGaA | |
| (2) Monsanto | |
| (3) Henkel KGaA | |
| (4) Haarmann & Reimer | |
| (5) BASF | |

Für Phase A wird das Pigment in Wasser eingerührt. Xanthan Gum wird unter Rühren langsam in die Suspension eingestreut und solange gerührt bis es gelöst ist. Die Phasen B und C werden nacheinander hinzugefügt und anschließend wird solange gerührt bis alles homogen verteilt ist.

Das Duschgel zeichnet sich durch intensive Interferenz-Glitzereffekte aus.

| Beispiel 4: - Eveliner Gel | |
|---|---|
| Phase A | |
| 15,00 % | Glimmerpigment der Teilchengröße 10-60 µm, beidseitig beschichtet mit TiO₂, SiO₂, TiO₂ (1) |
| 5,0 % | Glimmerpigment der Teilchengröße 10-60 µm beschichtet mit Fe₃O₄ (1) |
| 2,00 % | Ronasphere® (Silica) (1) |
| 0,40 % | Carbapol ETD 2001 (Carbomer) (2) |
| q.s. | Citric acid (1) |
| 60,00 % | Wasser |

| Phase B | |
|---|---|
| 4,0 % | Glycerin (1) |
| 0,90 % | Triethanolamine (1) |
| 2,00 % | Luviskol VA 64 Pulver (PVP/VA Copolymer) (3) |
| 0,20 % | Germaben II (Propylene Glycol, Diazolidinyl Urea, Methylparaben, Propylparaben) (4) |
| ad 100,00 % | Wasser |

| | |
|---|---|
| Bezugsquellen: (1) Merck KGaA | |
| (2) BF Goodrich | |
| (3) BASF | |
| (4) ISP Europe | |

Die Pigmente und Ronasphere® werden im Wasser der Phase A dispergiert. Mit einigen Tropfen Zitronensäurelösung wird der pH auf 3,8 eingestellt um die Viskosität zu vermindern. Carbopol wird unter Rühren eingestreut. Nach vollständiger Lösung wird die vorgelöste Phase B langsam eingerührt.

Das erhaltene Produkt zeichnet sich durch hohen Glanz und intensive Interforenzfarbe aus.

## Patentansprüche

1. Kosmetische Formulierung enthaltend ein Pigmentgemisch bestehend aus mindestens zwei Komponenten, wobei Komponente A Mehrschichtpigmente (Multischichtpigmente) auf Basis von Glimmer, SiO₂-Plättchen, Glas-Plättchen, Al₂O₃-Plättchen oder Polymerplättchen und Komponente B plättchenförmige, nadelförmige oder sphärische Farbmittel und/oder Füllstoffe sind.

2. Kosmetische Formulierung nach Anspruch 1, **dadurch gekennzeichnet, daß** Komponente A Mehrschichtpigmente auf Basis von SiO₂-Plättchen. Glimmerplättchen, Glasplättchen, Al₂O₃-Plättchen beschichtet mit alternierenden Schichten aus hoch- und niedrigbrechenden Metalloxiden sind.

3. Kosmetische Formulierung nach Anspruch 2, **dadurch gekennzeichnet, daß** es sich um natürliche oder synthetische Glimmerplättchen handelt.

4. Kosmetische Formulierung nach Anspruch 2 oder 3, **dadurch gekennzeichnet, daß** das Mehrschichtpigment eine hochbrechende Schicht aus Titandioxid, Zirkonoxid, Zinkoxid, Eisenoxid, Eisen-TitanOxid (Eisentitanate) und/oder Chromoxid enthält.

5. Kosmetische Formulierung nach Anspruch 4, **dadurch gekennzeichnet, daß** die hochbrechende Schicht aus TiO₂ und/oder Fe₂O₃ besteht.

6. Kosmetische Formulierung nach Anspruch 2 oder 3, **dadurch gekennzeichnet, daß** das Mehrschichtpigment als niedrigbrechende Schicht eine Schicht aus SiO₂, Al₂O₃, MgF₂ oder ein Polymer enthält

7. Kosmetische Formulierung nach einem der Ansprüche 1 bis 6 **dadurch gekennzeichnet, daß** das Mehrschichtpigment der Komponente A 2, 3, 4, 5, 6 oder 7 Schichten enthält.

8. Kosmetische Formulierung nach einem der Ansprüche 1 bis 7, **dadurch gekennzeichnet, daß** Komponente A ein Mehrschichtpigment enthält, das eine Fe₂O₃-, SiO₂-, Fe₂O₃-Beschichtung, eine TiO₂/Fe₂O₃-, SiO₂-, TiO₂/Fe₂O₃-Beschichtung oder eine TiO₂-, SiO₂-, TiO₂-Beschichtung aufweist.

9. Kosmetische Formulierung nach einem der Ansprüche 1 bis 8, **dadurch gekennzeichnet, daß** das Mehrschichtpigment der Komponente A ausgewählt ist aus der Gruppe
Substrat + Fe₂O₃-Schicht + SiO₂-Schicht + Fe₂O₃-Schicht
Substrat + Fe₂O₃-Schicht + SiO₂-Schicht + TiO₂-Schicht
Substrat + TiO₂-Schicht + SiO₂-Schicht + Fe₂O₃-Schicht
Substrat + TiO₂-Schicht + SiO₂-Schicht + TiO₂/Fe₂O₃-Schicht
Substrat + TiO₂/Fe₂O₃-Schicht + SiO₂-Schicht + TiO₂/Fe₂O₃-Schicht
Substrat + TiO₂-Schicht + SiO₂-Schicht + Cr₂O₃-Schicht.

10. Kosmetische Formulierung nach einem der Ansprüche 1 bis 9, **dadurch gekennzeichnet, daß** Komponente B Perlglanzpigmente, eingefärbte Glaspartikel, Ruß, organische Farbpigmente und/oder anorganische Farbpigmente sind,

11. Kosmetische Formulierung nach einem der Ansprüche 1 bis 10, **dadurch gekennzeichnet, daß** die organischen und anorganischen Farbpigmente und Farbstoffe natürlichen oder synthetischen Ursprungs sind.

12. Kosmetische Formulierung nach einem der Ansprüche 1 bis 11, **dadurch gekennzeichnet, daß** Komponente B Chromoxid, Ultramarin, sphärische SiO₂- oder TiO₂-Pigmente sind.

13. Kosmetische Formulierung nach einem der Ansprüche 1 bis 12, **dadurch gekennzeichnet, daß** das sphärische Farbmittel ausgewählt ist aus der Gruppe TiO₂, eingefärbtes SiO₂, CaSO₄, Eisenoxide, Chromoxide, Ruß, organische Farbpigmente, Anthrachinon-Pigmente, Chinacridon-Pigmente, Diketopyrrolopyrrol-Pigmente, Phthalocyanin-Pigmente, Azopigmente, Isoindolin-Pigmente.

14. Kosmetische Formulierung nach einem der Ansprüche 1 bis 11, **dadurch gekennzeichnet, daß** das nadelförmige Farbmittel ausgewählt ist aus der Gruppe BiOCl, eingefärbte Glasfasem, α-FeOOH, organische Farbpigmente, Azopigmente, β-Phthalocyanin Cl Blue 15,3, Cromophtalgelb 8GN, Irgalith Blau PD56, Azomethinkupferkomplex Cl Yellow 129, Irgazingelb 5GT.

15. Kosmetische Formulierung nach der Anspruch 10, **dadurch gekennzeichnet, daß** das Pengtanzpigment auf der Basis von plättchenförmigen, transparenten oder semitransparenten Substraten basiert.

16. Kosmetische Formulierung nach Anspruch 15, **dadurch gekennzeichnet, daß** Substrat Glimmer, Talkum, Sericit, Kaolin, aus Glas oder anderen silikatischen Materialien besteht.

17. Kosmetische Formulierung nach Anspruch 15 oder 16, **dadurch gekennzeichnet, daß** Substrat mit farbigen oder farblosen Metalloxiden beschichtet sind.

18. Kosmetische Formulierung nach einem der Ansprüche 15 bis 17, **dadurch gekennzeichnet, daß** das Substrat mit TiO₂, Titansuboxiden, Titanoxidnitriden, Fa₂O₃, Fe₃O₄, SnO₂, Cr₂O₃, ZnO, CuO, NiO oder anderen Metalloxiden allein oder in Mischung, in einer einheitlichen Schicht oder in aufeinanderfolgenden Schichten beschichtet ist.

19. Kosmetische Formulierung nach einem der Ansprüche 1 bis 18, **dadurch gekennzeichnet, daß** Komponente B ein TiO₂-Glimmer, Fe₂O₃-Glimmer und/oder TiO₂/Fe₂O₃-Glimmerpigment ist.

20. Kosmetische Formulierung nach einem der Ansprüche 1 bis 19, **dadurch gekennzeichnet, daß** Komponente A und Komponente 8 im Verhältnis 10:1 bis 1:10 gemischt sind.

21. Kosmetische Formulierung nach einem der Ansprüche 1 bis 20 für die dekorative und pflegende Kosmetik.

22. Kosmetische Formulierung nach einem der Ansprüche 1 bis 21 für Lippensfifte, Nagellacke, Lidschatten, Presspuder, Shampoos, lose Puder, Gele, Badezusätze, Zahnpasten.

23. Kosmetische Formulierung nach einem der Ansprüche 1 bis 22, **dadurch gekennzeichnet, daß** es das Pigmentgemisch in Mengen von 0,1 bis 70 Gew.% enthält.
